(19) Europäisches Patentamt
European Patent Office
Office européen des brevets

(11) **EP 4 620 457 B1**

(12) **EUROPEAN PATENT SPECIFICATION**

(45) Date of publication and mention
of the grant of the patent:
**08.07.2026 Bulletin 2026/28**

(21) Application number: **24190099.2**

(22) Date of filing: **22.07.2024**

(51) International Patent Classification (IPC):
*A61K 9/20* *(2006.01)*

(52) Cooperative Patent Classification (CPC):
**A61K 9/2068; A61K 9/2009; A61K 9/2013;
A61K 9/2018; A61K 9/205; A61K 9/2054**

(54) **SUSTAINED-RELEASE FORMULATION CONTAINING ACETONE EXTRACTED PRODUCT OF GAMBOGE RESIN**

FORMULIERUNG MIT VERZÖGERTER FREISETZUNG MIT ACETON EXTRAHIERTEM PRODUKT VON GAMBOGE-HARZ

FORMULATION À LIBÉRATION PROLONGÉE CONTENANT UN PRODUIT EXTRAIT À L'ACÉTONE DE RÉSINE DE GAMBOUGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **18.03.2024 TW 113110007**

(43) Date of publication of application:
**24.09.2025 Bulletin 2025/39**

(73) Proprietor: **Taiwan Sunpan Biotechnology
Development Co., Ltd.
Taoyuan City 33045 (TW)**

(72) Inventors:
• **LEE, Sen-Bin**
**33045 Taoyuan City (TW)**
• **LEE, Chia-Ling**
**33045 Taoyuan City (TW)**
• **LI, Yi-Ling**
**33045 Taoyuan City (TW)**
• **LI, Chiao-Ling**
**33045 Taoyuan City (TW)**

(74) Representative: **Gilani, Anwar
Venner Shipley LLP
406 Science Park
Milton Road
Cambridge CB4 0WW (GB)**

(56) References cited:
**CN-A- 101 244 097**

## Description

The present disclosure relates to a sustained-release formulation containing an acetone-extracted product of gamboge resin.

Gamboge resin is a gum-resin secreted by the plant of genus *Garcinia* of the family Guttiferae. It has been used as a source of vegetable dyes and pigments since ancient times. In some regions, such as India and Thailand, the gamboge resin is also used in folk medicine.

Gamboge (TENGHUANG in Hanyu Pinyin) is a kind of evergreen trees that grow in tropical regions. The main species grown in India is *Garcinia morella* Desv, whereas the main species grown in Thailand is *G. harburyi* Hook. Before the flowering period, the bark of the tree is cut open in a spiral shape about 2 meters from the ground to collect the exuding resin which is then subjected to heat-drying to result in a solidified gamboge resin.

According to records in traditional Chinese medicine (TCM) texts, gamboge is effective in combating inflammations, clearing away toxins, stopping bleeding, and killing worms. Since 1934, there have been a number of reports on the components of the gamboge resin. At present, it is known that many compounds can be isolated from the extract of the gamboge resin, including: morellin, morellic acid, gambogic acid, morellinol, isomorellin, isomorellic acid, isogambogic acid, isomorellinol, neogambogic acid, desoxymorellin, dihydroisomorellin, $\alpha$-guttiferin, $\beta$-guttiferin, gambogenic acid, desoxygambogenin, gambogellic acid, epigambogic acid, epiisogambogic acid, isogambogenic acid, and 30-hydroxygambogic acid, etc.

It has been reported that an acetone-extracted product of gamboge resin and the compounds obtained from such product have activities in inhibiting the growth of tumor/cancer cells, as well as analgesic and anti-inflammatory effects. For instance, US 7,138,428 B2 discloses an acetone-extracted product of gamboge resin and nine compounds which are further purified from such acetone-extracted product and which include a new compound, i.e., formoxanthone A, and eight known compounds, i.e., betulin, betulinic acid, morellic acid, isomorellic acid, gambogic acid, isogambogic acid, isomorellinol, and desoxymorellin. The acetone-extracted product and the nine purified compounds have been demonstrated to have effects in inhibiting the growth of tumor/cancer cells such as liver cancer cells (HepG2), lung cancer cells (A549), breast cancer cells (MCF-7), colon cancer cells (HT-29), leukemia cells (HL-60), and lymphoma cancer cells (U937).

US 2011/0305784 A1 discloses seventeen new compounds and five fractionated products obtained from an acetone-extracted product of gamboge resin. The seventeen new compounds and the five fractionated products have been demonstrated to have activities in inhibiting the growth of tumor/cancer cells. In addition, the acetone-extracted product and the five fractionated products obtained therefrom have been demonstrated to have analgesic and anti-inflammatory effects. CN 101244097 A discloses a slow release formulation containing gambogic acid.

In view of the aforesaid, there is still a need to develop a sustained-release formulation containing the acetone-extracted product of gamboge resin for medical applications.

Therefore, an object of the present disclosure is to provide a sustained-release formulation, which can alleviate at least one of the drawbacks of the prior art.

According to an aspect of the disclosure, there is provided a sustained-release formulation according to claim 1.

Other features and advantages of the disclosure will become apparent in the following detailed description of the embodiment(s) with reference to the accompanying drawings. It is noted that various features may not be drawn to scale.

FIG. 1 shows the high performance liquid chromatography (HPLC) spectrum of the acetone-extracted product TSB-9-W1 of the gamboge resin of Example 2, *infra.*
FIG. 2 is a graph showing the change in *in vitro* dissolution rate over time for each of the tablet of the present disclosure and the acetone-extracted product TSB-9-W1 of the gamboge resin of Example 4, *infra.*

For the purpose of this specification, it will be clearly understood that the word "comprising" means "including but not limited to", and that the word "comprises" has a corresponding meaning.

It is to be understood that, if any prior art publication is referred to herein, such reference does not constitute an admission that the publication forms a part of the common general knowledge in the art, in Taiwan or any other country.

Unless defined otherwise, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, $\pm$ 100% in some aspects, $\pm$ 50% in some aspects, $\pm$ 20% in some aspects, $\pm$ 10% in some aspects, $\pm$ 5% in some aspects, $\pm$1% in some aspects, $\pm$ 0.5% in some aspects, and $\pm$ 0.1% in some aspects from the specified amount, as such variations are appropriate to perform the disclosed methods or employ the disclosed compositions.

The present disclosure provides a sustained-release formulation, which includes, based on the total weight of the sustained-release formulation, 25.00 wt% to 31.25 wt% of an active pharmaceutical ingredient (API) including an acetone-extracted product of gamboge resin, 4 wt% to 25 wt% of sodium lauryl sulfate (SLS), 15.63 wt% to 23.44 wt% of hydroxypropyl methylcellulose, 6.25 wt% to 13.38 wt% of microcrystalline cellulose, 0.63 wt% to 1.56 wt% of silicon dioxide, 0.63 wt% to 1.56 wt% of magnesium stearate, 25.00 wt% to 40.13 wt% of lactose, and 5.00 wt% to 7.81 wt% of dextrose.

In an exemplary embodiment, the sustained-release formulation may include, based on the total weight of the sustained-release formulation, 31.25 wt% of the API, 5 wt% of the SLS, 18.75 wt% of the hydroxypropyl methylcellulose, 6.25 wt% of the microcrystalline cellulose, 1.56 wt% of silicon dioxide, 1.56 wt% of the magnesium stearate, 27.81 wt% of the lactose, and 7.81 wt% of the dextrose.

As used herein, the term "sustained-release formulation" can be used interchangeably with other terms such as "controlled-release formulation", and refers to any formulation that maintains a constant level of a drug in the blood by gradually releasing the drug over an extended period of time. In certain embodiments, the sustained-release formulation can gradually release the drug over a 12-hour to 72-hour time period. In an exemplary embodiment, the sustained-release formulation can gradually release the drug over a 24-hour time period.

As used herein, the term "active pharmaceutical ingredient" can be used interchangeably with other terms such as "active component" and "biologically active compound", and is understood to include any substance or material, or combination of substances and materials that is pharmacologically active and hence has therapeutic value.

According to the present disclosure, the active pharmaceutical ingredient (API) has a mean particle size of approximately 10 $\mu$m.

According to the present disclosure, the acetone-extracted product of the gamboge resin may be produced by the following steps a) to d).

In step a), the gamboge resin is pulverized into a powder, followed by extracting the pulverized powder with acetone, so as to obtain a first extract.

In step b), the first extract is subjected to an ultrasonic treatment, so as to obtain a second extract.

In step c), the second extract is subjected to a filtration treatment, so as to obtain a filtrate.

In step d), the filtrate is subjected to a concentration treatment to remove the acetone.

In certain embodiments, in step a), a weight-to-volume ratio (g/mL) of the pulverized powder to the acetone ranges from 1:2 to 1:3.

In certain embodiments, in step b), the ultrasonic treatment is conducted at a temperature ranging from 20°C to 35°C.

In certain embodiments, in step c), the filtration treatment is conducted by using a filter paper with a pore size ranging from 3 $\mu$m to 6 $\mu$m.

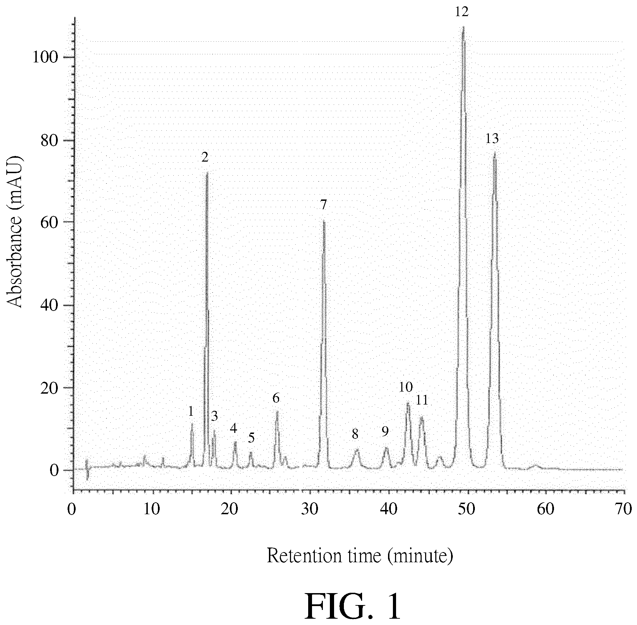

According to the present disclosure, when the acetone-extracted product of the gamboge resin is subjected to high performance liquid chromatography (HPLC) analysis, an HPLC spectrum as shown in FIG. 1 is obtained.

According to the present disclosure, the sustained-release formulation may be formulated into a dosage form suitable for oral administration using technology well-known to those skilled in the art. Examples of the dosage form may include, but are not limited to, a tablet (e.g., a coated tablet), a granule, a powder, a capsule (e.g., a coated capsule and a pellet-filled capsule), a pellet, and a pill. In an exemplary embodiment, the sustained-release formulation is formulated into a tablet.

According to the present disclosure, the sustained-release formulation may be formulated into a tablet using direct compression or dry granulation well-known to those skilled in the art.

The disclosure will be further described by way of the following examples. However, it should be understood that the following examples are solely intended for the purpose of illustration and should not be construed as limiting the disclosure in practice.

**EXAMPLES**

*General Procedures:*

**Example 1. Preparation of acetone-extracted product TSB-9-W1 of gamboge resin**

The acetone-extracted product TSB-9-W1 (i.e., an active pharmaceutical ingredient (API)) of the gamboge resin was prepared by a method slightly modified from that described in Example 1 of US 7,138,428 B2. First, the gamboge resin was pulverized into a powder, followed by sieving the pulverized powder using a sieve (mesh No. 40, Manufacturer: Kuang Yang) with a mesh number of 40 mesh, and then impregnating and extracting the sieved powder with acetone (a weight-to-volume ratio (g/mL) of the sieved powder to the acetone was 1:3) at room temperature for 12 hours, so as to obtain a first extract. Next, the first extract was subjected to ultrasonic oscillation using an ultrasonic extraction equipment (Manufacturer: Taiwan Supercritical Technology Co., Ltd., Model no.: ES-600N) at an initial water temperature ranging from 20°C to 35°C for 1 hour, so as to obtain a second extract. The second extract was then subjected to a filtration treatment using a filter paper NO. 1 (Manufacturer: Advantec) with a pore size of 6 μm, so as to obtain a residue on the filter paper and a filtrate. Subsequently, the thus obtained residue was subjected to the aforesaid acetone impregnation, ultrasonic oscillation, and filtration treatment conducted in sequence four times. During the last four times of the acetone impregnations, a weight-to-volume ratio (g/mL) of the residue to the acetone was 1:2. Afterwards, all of the resultant filtrate was collected and was then subjected to a concentration treatment using a rotary evaporator (Manufacturer: Hocon-Engineering Enterprise Co., Ltd., Model no.: 16A8-19) to remove most of the acetone therein, followed by conducting a vacuum drying process using a vacuum oven (Manufacturer: Dengyng Instrument Co., Ltd., Model no.: DOV-40) at a temperature of 40°C, so as to obtain a dried extract. Thereafter, the dried extract was pulverized using a floor standing pulverizer (Manufacturer: Yu Chi Ling Co., Ltd., Model no.: FM) equipped with a sieve with a pore size of 0.2 mm, so as to obtain a dried powder of the acetone-extracted product TSB-9-W1 of the gamboge resin.

**Example 2. High performance liquid chromatography (HPLC) analysis** *Experimental Procedures:*

The dried powder of the acetone-extracted product TSB-9-W1 of the gamboge resin obtained in Example 1 was dissolved in an appropriate amount of acetonitrile, thereby obtaining a test sample of the acetone-extracted product TSB-9-W1 having a concentration of 1 mg/mL.

The test sample was subjected to high performance liquid chromatography (HPLC) analysis using technology well-known to those skilled in the art. The operating parameters and conditions for performing HPLC are summarized in Table 1 below.

Table 1

| | |
|---|---|
| HPLC instrument | Chromaster HPLC system (Manufacturer: Hitachi, Model no.: CM-5000 series) equipped with a pump (Manufacturer: Hitachi, Model no.:5110) and a diode array detector (Manufacturer: Hitachi, Model no.:5430) |
| Type of chromatography column | Luna 3 μm C8(2) 100 Å Column (Manufacturer: Phenomenex) |
| Size of chromatography column | Length: 150 mm; inner diameter: 4.6 mm |
| Temperature of chromatography column | 35°C |
| Injection volume of test sample | 10 μL |
| Detection wavelength | 360 nm |
| Mobile phase | Acetonitrile / 0.05% trifluoroacetic acid (TFA) solution (65:35, v/v) |
| Flow rate of test sample | 0.75 mL/min |

*Results:*

FIG. 1 shows the HPLC spectrum of the acetone-extracted product TSB-9-W1 of the gamboge resin. As shown in FIG. 1, there were thirteen main peaks (i.e., peaks 1 to 13) during a retention time from 0 minutes to 70 minutes, indicating that there were thirteen major components in the acetone-extracted product TSB-9-W1 of the gamboge resin.

**Example 3. Preparation of hydrophobic long-acting tablet containing acetone-extracted product TSB-9-W1**

*Experimental Materials:*

The ingredients and the amounts thereof for making the hydrophobic long-acting tablet (serving as a drug product) containing the acetone-extracted product TSB-9-W1 are shown in Table 2 below.

Table 2

| Ingredient | Amount | |
|---|---|---|
| | mg/tablet | wt% |
| TSB-9-W1 | 200 | 31.25 |
| Sodium lauryl sulfate (SLS, Manufacturer: BASF Co., Ltd.) | 32 | 5.00 |
| Hydroxypropyl methylcellulose K4M (Manufacturer: Colorcon Asia Pacific Pte Ltd.) | 120 | 18.75 |
| Microcrystalline cellulose 102 (Manufacturer: Mingtai Chemical Co., Ltd.) | 40 | 6.25 |
| Silicon dioxide (Manufacturer: PQ Corporation) | 10 | 1.56 |
| Magnesium stearate (Manufacturer: Peter Greven GmbH & Co. Kg) | 10 | 1.56 |
| Lactose (i.e., Lactose monohydrate, Manufacturer: DFE Pharma GmbH & Co. Kg) | 178 | 27.81 |
| Dextrose (Manufacturer: Weifang Shengtai Medicine Co., Ltd.) | 50 | 7.81 |
| Total weight per tablet was 640 mg | | |

*Experimental Procedures:*

First, the acetone-extracted product TSB-9-W1 obtained in Example 1 was pulverized, so that the acetone-extracted product TSB-9-W1 (serving as an API) has a mean particle size of 10 μm. A respective one of SLS, hydroxypropyl methylcellulose K4M, microcrystalline cellulose 102, silicon dioxide, magnesium stearate, lactose, and dextrose was sieved using a sieve (mesh No. 40, Manufacturer: Kuang Yang) with a mesh number of 40 mesh. Next, the acetone-extracted product TSB-9-W1 with a mean particle size of 10 μm, the sieved SLS, the sieved hydroxypropyl methylcellulose K4M, the sieved microcrystalline cellulose 102, the sieved lactose, and the sieved dextrose were mixed together to obtain a first mixture, followed by compressing the first mixture into a lump using a rotary tableting machine (Manufacturer: Chuang Pao Special Precision Industry Co., Ltd., Model no.: CB-747). Thereafter, the lump was pulverized into a powder, followed by sieving the pulverized powder using a sieve (mesh No. 20, Manufacturer: Kuang Yang) with a mesh number of 20 mesh. The resultant sieved powder was mixed with the sieved silicon dioxide and the sieved magnesium stearate, so as to obtain a second mixture. The second mixture was then compressed into tablets using the rotary tableting machine. Each of the resultant tablets had dimensions of 0.9 cm (width) × 1.6 cm (length) × 0.515 cm (height), and a weight of 640 mg.

**Example 4. Dissolution test of tablet containing acetone-extracted product TSB-9-W1**

The tablet (i.e., sustained-release formulation) obtained in Example 3 was subjected to a dissolution test, so as to evaluate the *in vitro* release profile for the acetone-extracted product TSB-9-W1 released from the tablet. For the purpose of comparison, the acetone-extracted product TSB-9-W1 obtained in Example 1 was subjected to the same dissolution test.

*Experimental Procedures:*

The *in vitro* dissolution rates of three tablets each containing the acetone-extracted product TSB-9-W1 (i.e., API) were evaluated using a USP dissolution apparatus II machine (Manufacturer: Tianda Tianfa Technology Co., Ltd, Model no.: RC806D). First, a respective one of the three tablets was placed in 900 mL of 50 mM sodium phosphate monobasic solution (pH 6.8) containing 0.25% SLS, and agitated at a paddle speed of 50 rpm at 37 ± 0.5°C for 24 hours. During the agitating period, 5 mL of the resultant dissolution solution for the respective one of the three tablets was collected at irregular intervals (approximately every 0.5 hours to 3 hours), and was subsequently filtered using a filter (Manufacturer: Pall Corporation) with a porosity of 0.45 μm, so as to collect a filtrate to serve as a test solution. The thus obtained test solution was then subjected to determination of absorbance at a wavelength of 360 nm ($A_{360}$) using a UV-VIS spectrophotometer (Manufacturer: Shishin Technology Co., Ltd, Model no.: SP-8001). In addition, 200 mg of the acetone-extracted product TSB-9-W1 was dissolved in 900 mL of 50 mM sodium phosphate monobasic solution (pH 6.8) containing 0.25% SLS, and then was subjected to the same dissolution test.

Next, the thus obtained $A_{360}$ value of a respective one of the acetone-extracted product TSB-9-W1 and the three tablets each containing the acetone-extracted product TSB-9-W1 was converted to their concentrations of the acetone-extracted product TSB-9-W1 (i.e., API) expressed in mg/mL according to standard curves prepared in advance using standards with different known concentrations of the acetone-extracted product TSB-9-W1 (i.e., API) relative to their own $A_{360}$ values, followed by calculating the total dissolution amount (mg) of TSB-9-W1 (i.e., API) for the respective one of the acetone-extracted product TSB-9-W1 and the three tablets of the present disclosure at different time points.

The dissolution rate (%) of the respective one of the acetone-extracted product TSB-9-W1 and the three tablets each containing the acetone-extracted product TSB-9-W1 of the present disclosure was calculated by substituting the total dissolution amount of the API and the initial weight of the API (i.e., 200 mg) into the following Equation (1):

$$A = (B/C) \times 100 \qquad\qquad (1)$$

where

A=dissolution rate (%)
B=total dissolution amount (mg) of the API for each of the tablets of the disclosure and the acetone-extracted product TSB-9-W1
C=initial weight of the API for each of the tablets of the disclosure and the acetone-extracted product TSB-9-W1 (i.e., 200 mg)

The dissolution rates thus obtained at each time point of the three tablets were averaged to determine the average dissolution rate at each time point for the tablet of the present disclosure. The average dissolution rate determined at each time point for the tablet (i.e., the sustained-release formulation) of the present disclosure and the dissolution rate determined at each time point for the acetone-extracted product TSB-9-W1 were plotted against time to create a corresponding dissolution profile.

### Results:

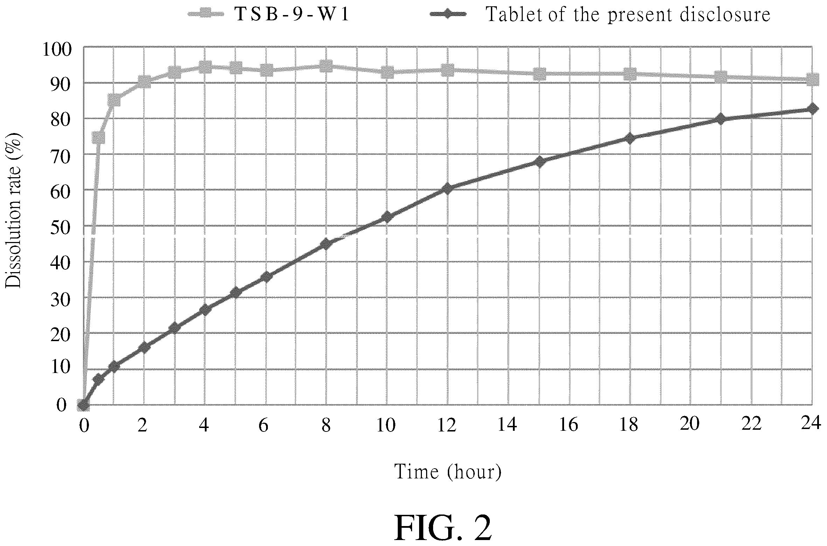

FIG. 2 shows the change in *in vitro* dissolution rate over time for each of the tablet (i.e., the sustained-release formulation) of the present disclosure and the acetone-extracted product TSB-9-W1. As shown in FIG. 2, the dissolution rates of the acetone-extracted product TSB-9-W1 were not lower than 90% after 2 hours from the start of the dissolution test, while the average dissolution rate of the tablet (i.e., the sustained-release formulation) of the present disclosure was only 16.1% at the 2nd hour from the start of the dissolution test. Moreover, the dissolution rate of the tablet (i.e., the sustained-release formulation) of the present disclosure increased slowly over time, reaching only approximately 80% by the end of the 24-hour dissolution test. These results demonstrate that the tablet (i.e., the sustained-release formulation) of the present disclosure can effectively and sustainably release the acetone-extracted product TSB-9-W1 (i.e., API).

Summarizing the above test results, it is clear that the sustained-release formulation of the present disclosure can effectively and sustainably release the acetone-extracted product of the gamboge resin.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, the one or more features may be singled out and practiced alone without the another one or more features or specific details. It should be further noted that one or more features or specific details from one embodiment may be practiced together with one or more features or specific details from another embodiment, where appropriate, in the practice of the disclosure.

## Claims

1. A sustained-release formulation **characterized by**, based on the total weight of the sustained-release formulation:

25.00 wt% to 31.25 wt% of an active pharmaceutical ingredient, API, including an acetone-extracted product of gamboge resin;
4 wt% to 25 wt% of sodium lauryl sulfate, SLS;
15.63 wt% to 23.44 wt% of hydroxypropyl methylcellulose;
6.25 wt% to 13.38 wt% of microcrystalline cellulose;
0.63 wt% to 1.56 wt% of silicon dioxide;
0.63 wt% to 1.56 wt% of magnesium stearate;
25.00 wt% to 40.13 wt% of lactose; and
5.00 wt% to 7.81 wt% of dextrose.

2. The sustained-release formulation as claimed in claim 1, wherein the acetone-extracted product of the gamboge resin is produced by the steps of:

a) pulverizing the gamboge resin into a powder, followed by extracting the pulverized powder with acetone, so as to obtain a first extract;
b) subjecting the first extract to an ultrasonic treatment, so as to obtain a second extract;
c) subjecting the second extract to a filtration treatment, so as to obtain a filtrate; and
d) subjecting the filtrate to a concentration treatment to remove the acetone.

3. The sustained-release formulation as claimed in claim 2, wherein in step a), a weight-to-volume ratio (g/mL) of the pulverized powder to the acetone ranges from 1:2 to 1:3.

4. The sustained-release formulation as claimed in claim 2, wherein in step b), the ultrasonic treatment is conducted at a temperature ranging from 20°C to 35°C.

5. The sustained-release formulation as claimed in claim 2, wherein in step c), the filtration treatment is conducted by using a filter paper with a pore size ranging from 3 $\mu$m to 6 $\mu$m.

6. The sustained-release formulation as claimed in any one of claims 1 to 5, wherein the acetone-extracted product of the gamboge resin has an high performance liquid chromatography, HPLC, spectrum as shown in FIG. 1.

**Patentansprüche**

1. Formulierung mit verzögerter Freisetzung, **gekennzeichnet durch**, basierend auf dem Gesamtgewicht der Formulierung mit verzögerter Freisetzung:

25,00 Gew.-% bis 31,25 Gew.-% eines aktiven pharmazeutischen Inhaltsstoffes, API, beinhaltend ein Aceton extrahiertes Produkt von Gamboge-Harz;
4 Gew.-% bis 25 Gew.-% Natriumlaurylsulfat, SLS;
15,63 Gew.-% bis 23,44 Gew.-% Hydroxypropylmethylcellulose;
6,25 Gew.-% bis 13,38 Gew.-% mikrokristalline Cellulose;
0,63 Gew.-% bis 1,56 Gew.-% Siliziumdioxid;
0,63 Gew.-% bis 1,56 Gew.-% Magnesiumstearat;
25,00 Gew.-% bis 40,13 Gew.-% Lactose; und
5,00 Gew.-% bis 7,81 Gew.-% Dextrose.

2. Formulierung mit verzögerter Freisetzung nach Anspruch 1, wobei das Aceton extrahierte Produkt des Gamboge-Harzes durch die folgenden Schritte produziert wird:

a) Pulverisieren des Gamboge-Harzes zu einem Pulver, gefolgt von Extrahieren des pulverisierten Pulvers mit Aceton, um einen ersten Extrakt zu erhalten;
b) Unterziehen des ersten Extrakts einer Ultraschallbehandlung, um einen zweiten Extrakt zu erhalten;
c) Unterziehen des zweiten Extrakts einer Filtrationsbehandlung, um ein Filtrat zu erhalten; und
d) Unterziehen des Filtrats einer Konzentrationsbehandlung, um das Aceton zu entfernen.

3. Formulierung mit verzögerter Freisetzung nach Anspruch 2, wobei in Schritt a) ein Gewichtszu-Volumen-Verhältnis (g/ml) des pulverisierten Pulvers zu dem Aceton von 1:2 bis 1:3 reicht.

**4.** Formulierung mit verzögerter Freisetzung nach Anspruch 2, wobei in Schritt b) die Ultraschallbehandlung bei einer Temperatur durchgeführt wird, die von 20 °C bis 35 °C reicht.

**5.** Formulierung mit verzögerter Freisetzung nach Anspruch 2, wobei in Schritt c) die Filtrationsbehandlung durch Verwenden eines Filterpapiers mit einer Porengröße durchgeführt wird, die von 3 $\mu$m bis 6 $\mu$m reicht.

**6.** Formulierung mit verzögerter Freisetzung nach einem der Ansprüche 1 bis 5, wobei das Aceton extrahierte Produkt des Gamboge-Harzes ein Hochleistungs-Flüssigkeitschromatographie-Spektrum, HPLC-Spektrum, wie gezeigt in FIG. 1 aufweist.

**Revendications**

**1.** Formulation à libération prolongée **caractérisée par**, sur la base du poids total de la formulation à libération prolongée :

25,00 % en poids à 31,25 % en poids d'un principe actif pharmaceutique, API, comprenant un produit extrait à l'acétone de résine de gambouge ;
4 % en poids à 25 % en poids de laurylsulfate de sodium, SLS ;
15,63 % en poids à 23,44 % en poids d'hydroxypropylméthylcellulose ;
6,25 % en poids à 13,38 % en poids de cellulose microcristalline ;
0,63 % en poids à 1,56 % en poids de dioxyde de silicium ;
0,63 % en poids à 1,56 % en poids de stéarate de magnésium ;
25,00 % en poids à 40,13 % en poids de lactose ; et
5,00 % en poids à 7,81 % en poids de dextrose.

**2.** Formulation à libération prolongée selon la revendication 1, dans laquelle le produit extrait à l'acétone de résine de gambouge est produit par les étapes de :

a) pulvérisation de la résine de gambouge en une poudre, suivie par l'extraction de la poudre pulvérisée avec de l'acétone, de façon à obtenir un premier extrait ;
b) soumission du premier extrait à un traitement par ultrasons, de façon à obtenir un deuxième extrait ;
c) soumission du deuxième extrait à un traitement de filtration, de façon à obtenir un filtrat ; et
d) soumission du filtrat à un traitement de concentration pour éliminer l'acétone.

**3.** Formulation à libération prolongée selon la revendication 2, dans laquelle, à l'étape a), un rapport poids/volume (g/mL) de la poudre pulvérisée à l'acétone est compris entre 1:2 et 1:3.

**4.** Formulation à libération prolongée selon la revendication 2, dans laquelle à l'étape b), le traitement par ultrasons est effectué à une température comprise entre 20° C et 35° C.

**5.** Formulation à libération prolongée selon la revendication 2, dans laquelle, à l'étape c), le traitement de filtration est effectué en utilisant un papier filtre avec une taille de pores comprise entre 3 $\mu$m et 6 $\mu$m.

**6.** Formulation à libération prolongée selon l'une quelconque des revendications 1 à 5, dans laquelle le produit extrait à l'acétone de la résine de gambouge a un spectre de chromatographie liquide haute performance, HPLC, tel que représenté sur figure 1.

FIG. 1

FIG. 2

**EP 4 620 457 B1**

**REFERENCES CITED IN THE DESCRIPTION**

**Patent documents cited in the description**

- US 7138428 B2
- US 20110305784 A1
- CN 101244097 A